# EUROPÄISCHE PATENTSCHRIFT

(11) **EP 3 025 741 B1**
(45) Veröffentlichungstag und Bekanntmachung des Hinweises auf die Patenterteilung: **21.02.2018**
(21) Anmeldenummer: 15003318.1
(22) Anmeldetag: 21.11.2015
(51) Int. Cl.: A61B 1/12, A61B 90/70, A61M 5/14

(54) **ANORDNUNG ZUR SPEICHERUNG UND VERABREICHUNG VON SPÜLLÖSUNGEN**
DEVICE FOR STORING AND ADMINISTRATION OF FLUSH SOLUTIONS
SYSTÈME DE STOCKAGE ET DE DISTRIBUTION DE SOLUTIONS DE RINÇAGE

(30) Priorität: 25.11.2014 DE 102014017402
(43) Veröffentlichungstag der Anmeldung: 01.06.2016
(73) Patentinhaber: Fidica GmbH & Co. KG, 63877 Sailauf (DE)
(72) Erfinder: Völker, Manfred, 63825 Blankenbach (DE)
(74) Vertreter: Flosdorff, Jürgen

(56) Entgegenhaltungen:
- EP-A2- 0 861 675
- JP-A- 2011 177 291
- US-A1- 2007 112 303

## Beschreibung

Die Erfindung betrifft eine Anordnung zur Speicherung und Verabreichung von Spüllösungen, vorzugsweise im Medizinbereich wie für allgemeine und endoskopische Operationen, mit einem Spüllösungsbeutel, der in einem starren Behälter angeordnet ist.
Ziel dieser Entwicklung ist die Aufbereitung, Speicherung und mobile Verabreichung vor Ort hergestellter Spüllösungen. Ein mobiler Flüssigkeitsspeicher der mit einem sterilen Einmalartikel bestückt wird, eine exakte Volumenbestimmung und eine sterile Entnahme und Verabreichung sollen eine einfache und kostengünstige Anwendung ermöglichen.
Dabei können sowohl Spüllösungen für endoskopische und allgemein chirurgische Operationen, z.B. Gynäkologie, Urologie, Arthroskopie durch die Verwendung von Purisole-, Ringer-, Kochsalzkonzentraten, als auch Lösungen für therapeutische Anwendungen hergestellt werden.
Eine Anwendung dieser Entwicklung für andere Bereiche wie z.B. für die Veterinärmedizin, im Labor oder in der Biologie und in der Pharmazie als hochreine Spülflüssigkeit oder auch Ansatzmedium zur Herstellung von Medikamenten, Zellkulturen und dergleichen ist vorstellbar und praktikabel.
Der Erfindung liegt insbesondere die Aufgabe zugrunde, eine Anordnung zur Speicherung und Verabreichung von Spüllösungen anzugeben, die hohe Hygieneanforderungen erfüllt und eine sterile Entnahme und einfache Bedienung und Verabreichung der Spülflüssigkeit ermöglicht.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Medizinische Spüllösungen werden in der Regel aus destilliertem Wasser als Grundstoff, der zentral hergestellt wird, in einem zentralen Produktionsprozess zu Spüllösungen weiterverarbeitet und mit erheblichen Logistikkosten zum Anwendungsort gebracht.

Für den medizinischen Einsatz werden beispielsweise industriell hergestellte Spüllösungen mit Volumen 3l, 5l, 10l dem Krankenhaus zur Verfügung gestellt und mit großem innerbetrieblichem, personellem Logistikeinsatz zwischen- u. endgelagert.

Für die Dauer der Operation bzw. Untersuchung reichen diese Beutelvolumina beispielhaft für eine Blasenoperation mit ca. 60 l Spülflüssigkeit nicht aus, sodass ein Springer außerhalb des zentralen OP-Bereiches verfügbar sein muss, um die Beutel bereit zu stellen, aufzuwärmen und zuzureichen.

Die Verabreichung erfolgt teils gravimetrisch oder auch mit Beutel-Druckmanschetten. Zusätzlich sind oft teure Einmalartikel wie beispielsweise Pumpensegmente oder auch für die Beutelwärmer erforderlich.
Ein erheblicher Nachteil z.B. bei endoskopischen Untersuchungen ist die mangelnde Sicht durch flotierendes Gewebe oder pulsierende Spülflüssigkeit, weil beispielsweise der erforderliche Spülflüssigkeitsdruck zwischen 0,1 bar bis 0,3 bar nicht konstant gehalten wird.
Zur Verbesserung der Wundhygiene ist großzügig zu spülen. Dies verursacht personelle- und auch Materialkosten.
Die regulativen und normativen Anforderungen an die Qualität des Grundstoffs Wassers sind dabei so hoch, dass es bisher nicht möglich ist, vor Ort, z.B. im Krankenhaus, verifizierbare medizinische Spüllösungen auf Bedarf herzustellen. Zum einen sind dies die hohen mikrobiologischen- und zum anderen die erforderlichen chemischen Anforderungen an den Grundstoff Wasser, die einer verifizier- und nachweisbaren normativ geforderten Qualität der vor Ort bedarfsgesteuerten Herstellung entgegenstehen.

Die dezentrale Herstellung von medizinischen Spüllösungen durch Krankenhauspersonal erfordert sichere Abläufe sowohl in der Bedienung als auch in der Zuverlässigkeit der Technik hinsichtlich der Spüllösungsqualität.

Notwendige Verbesserungen, Zweck und Ziel dieser Erfindung sind deshalb die kostengünstige, anwenderfreundliche vor-Ort-Herstellung einer Spüllösung mit geringem Personaleinsatz und einem der Untersuchung bzw. auch mehreren Operationen entsprechenden Spülvolumen.
Eine besondere Bedeutung kommt der unterbrechungsfreien Verabreichung ohne zusätzlichen personellen Mehraufwand unter Einhaltung von Anwendungstemperatur und Hygiene der Lösung zu.

Dabei sollen eine raumsparende Technik zur Herstellung der Spüllösung und ein mobiler Spüllösungsbehälter zum Einsatz kommen, der die wesentlichen Komponenten für hohe Hygiene, Sicherheit, einfache Bedienung und einen konstanten Fluss und Druck zur Verabreichung der Spülflüssigkeit enthält.

Restmengen sollen einfach zu entsorgen sein.

Eine hohe Verfügbarkeit der Geräte bei allen Mess- und Überwachungsaufgaben hinsichtlich ihrer Eigensicherheit und eine nur entfernte Ausfallwahrscheinlichkeit ist wichtig, um unter allen Umständen eine katastrophale Auswirkung für den Patienten zu vermeiden, bzw. die Qualität oder auch Toxizität der erzeugten Flüssigkeit einwandfrei in den zugesicherten Akzeptanzkriterien zu überwachen.

Diese Aufgabe wird erfindungsgemäß wirkungsvoll dadurch gelöst, dass zur Herstellung der Spüllösung die Kombination einer Umkehrosmosemembrane und zweier weiterer Filterstufen beispielsweise Ultra-oder Sterilfilter, bevorzugt als Kapillarmembran, genutzt werden.
Diese Filterkombination und weitere Bestandteile werden nachfolgend als Füllstation bezeichnet.

Zur Herstellung von beispielsweise ca. 60 I gebrauchsfertiger Purisolelösung sind ca. 561 sterilfiltriertes Permeat mit ca. 3,6l hochkonzentriertem Purisolekonzentrat proportional so zu verdünnen bzw. zu mischen, dass die entstandene Spüllösung ohne weitere Prüfungen zur intra- und postoperativen Blasenspülung zur Anwendung kommen kann.

Die vorgenannte Spüllösung steht stellvertretend z.B. für Ringer -, und/oder andere Natriumchloridlösungen, die insbesondere im Bereich der Chirurgie, aber auch in andren medizinischen bzw. genannten Bereichen eingesetzt werden können. Wobei die Konzentrate und deren Mischungsverhältnisse den jeweiligen Anwendungen anzupassen sind.

Das beschriebene Verfahren und die eingesetzten Komponenten und Volumen sind jedoch nicht darauf reduziert. Bedingt durch die hochreinen Wirkmittel, das exakte Mischen und Verdünnen ist eine große Bandbreite von Spüllösungen herstellbar.

Vorteilhafterweise wird durch die hohe Konzentration auch das Keimwachstum des Konzentrates nahezu verhindert.

Zur Aufbereitung der Spüllösung wird das Konzentratbehältnis das vorteilhaft als Beutel ausgeführt ist an vorbereitete Aufnahmen der Füllstationskonzentratwaage gehängt und der Mischvorgang eingeleitet. Dabei wird zunächst durch das bekannte Beutelgewicht die Waage verifiziert.

Die Füllstationsseitigen Anschlüsse des Konzentrats- als auch des noch zu beschreibenden Spüllösungsbeutels werden an selbstreinigende, verwechslungssichere Anschluss-Konnektoren der Füllstation, die in dieser Anmeldung beispielsweise als Klappenlösungen ausgeführt sind, aber auch geräteseitig als flexible Schlauchleitung ausgeführt sein können, durch den Anwender herbeigeführt.

Mit großem Vorteil wird ein fahrbarer Spüllösungsbehälter, der vorzugsweise als Druckbehälter ausgebildet ist, mit einem einlegbaren sterilen Spülflüssigkeitsbeutel bestückt, der mit einem entsprechend großen Volumen befüllt wird.

Der Spüllösungsbeutel beinhaltet einen unlösbaren Anschlusskonnektor, der durch den verriegelbaren Deckel des Druckbehälters durchgesteckt und fixiert werden kann. Der Anschlusskonnektor kann mit weiterführenden flexiblen Schlauchleitungen versehen werden, die als Füll- bzw. Transferleitungen ausgebildet sind. Wobei der Konnektor mit Vorteil auch nur einen Schlauch ausgeführt sein kann der wahlweise und in Abhängigkeit der Sterilitätsanforderungen sowohl als Füll- als auch als Transferschlauch zu verwenden ist.

Zur Verabreichung der Spülflüssigkeit am Anwendungsort kann an den Transferanschluss des Spüllösungskonnektors ein Überleitsystem beispielsweise mit einem Endoskopiesystem verbunden werden. Ein Anschluss an andre in der Chirurgie üblichen Systemen beispielweise an Spül-Saugsysteme ist ebenfalls praktikabel und möglich.

Gelöst wurde die Aufgabe der einfachen Bedienung und der Verabreichung mit konstantem Spülfluss und -Druck, indem Druckgas (Luft) entweder vorzugsweise in den Druckbehälter oder auch wahlweise direkt in den Spülflüssigkeitsbeutel eingeleitet wird.

Dabei wird mit Vorteil die Druckgasregelung und Überwachung innerhalb des fahrbaren Spüllösungsbehälters eingerichtet. Druckgaserzeugung und Einspeisung können beispielsweise durch eine hauseigene Quelle, aber auch geräteseitig erzeugt werden.

Die Propörtionierung von Konzentrat und Permeat erfolgt mittels einer Konzentrat- und einer Spüllösungsbehälterwaage, wobei die Konzentratwaage in der Füllstation mit jedem Anhängen des befüllten Konzentratbehälters verfiziert wird.

Dazu beinhaltet der fahrbare Spüllösungsbehälter mit Vorteil eine Waage, die den Füllzustand überwacht und die aus Sicherheitsgründen mittels Referenzgewicht automatisch zu testen ist.

Zur Homogenisierung und Temperierung wird hochreines bzw. annähernd steriles Permeat erwärmt und mit zudosiertem Konzentrat in einem Mischblock gemischt.

Vor Einleitung in einen sterilen Spüllösungsbehälter-/Beutel erfolgte eine zweite Sterilfiltrierung der gemischten Lösung.

Die Reinigung des Systems bzw. Keimprävention und Reduktion werden durch die Kombination aus einem gering toxischen, auf Zitrat basierenden Desinfektions- u. Reinigungsmittel mit einer Wassererwärmung ausgeführt. Wobei sowohl die Primär-, als auch die Sekundärseite der Umkehrosmose getrennt voneinander mittels einer zusätzlichen Pumpe auch ohne transmembranen Fluss zu desinfizieren bzw. zu reinigen sind.

Prinzipiell werden dabei alle prozessrelevanten Daten sowohl vom Betriebs- als auch vom Schutzrechner erfasst und ggf. berechnet. Die Messergebnisse werden vom Betriebs- zum Schutzrechner und umgekehrt gesendet. Jeder Rechner vergleicht dabei die Messergebnisse mit den eigenen und gibt eine Bestätigung zurück.

Die Daten werden nach der Bestätigung von Betriebs- und Schutzrechner zusammen mit einer Prüfsumme in den Trenddatenspeicher geschrieben, der vorzugsweise als Eprom, aber auch als anderes Speichermedium ausgebildet sein kann.

Die Elektronik des fahrbaren Spüllösungsbehälters kann mittels aufladbaren Akkus betrieben werden, an der Anzeige des fahrbaren Spüllösungsbehälter werden alle erforderlichen Parameter als auch deren Abweichungen wie beispielsweise Gewicht, Temperatur und Behälterdruck angezeigt.

Indem ein drahtloser Datenaustausch zwischen Füllstation und fahrbarem Spüllösungsbehälter hergestellt wird, erfolgt beispielsweise die Überwachung der Befüllung, der Proportionalität und der Temperatur.

Weitere Einzelheiten und Vorteile sind in den nachfolgend dargestellten Figuren beschrieben.

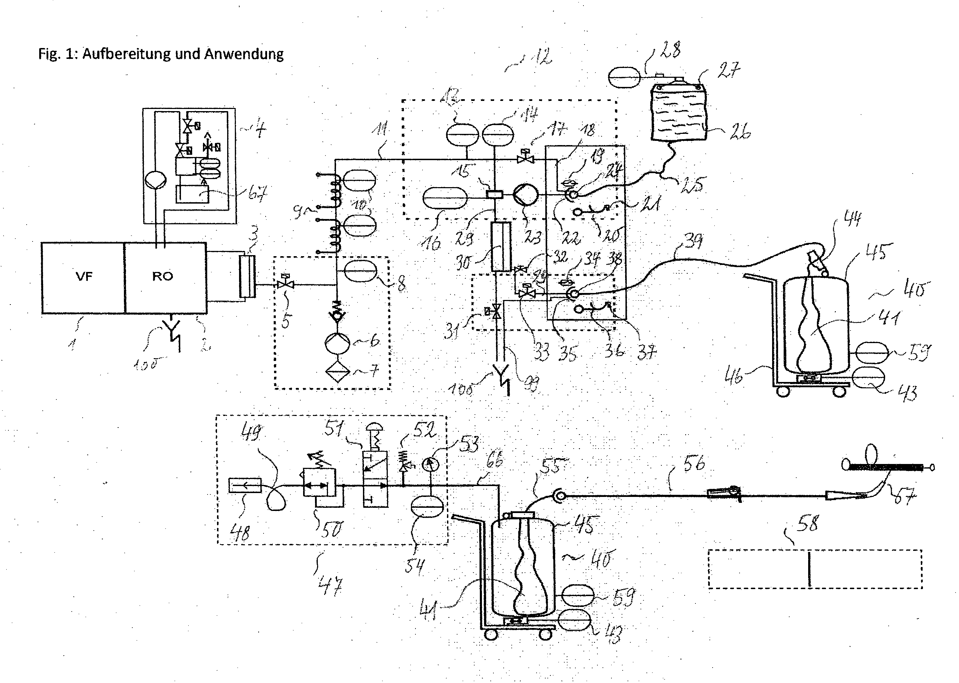

Figur 1 zeigt die gesamte Aufbereitung bis hin zur Anwendung. Die aufzubereitende Flüssigkeit kann beispielsweise über eine optionale Vorfiltration (1), die als Partikel- und oder weiterer Filterstufen zur Eliminierung von Härtebildnern und Chlor ausgebildet sein kann, zur RO-Anlage (2) weitergeleitet.

Zur Eliminierung mikrobiologischer Kontamination beinhaltet die RO (2) beispielsweise eine Desinfektionseinheit (4) mit der, ohne Zutun des Anwenders, eine chemothermische Desinfektion durchführbar ist. Kanister (67) beinhaltet das Desinfektions-/Reinigungsmittel das mit Vorteil als citrathaltige Lösung verwendet wird. Die weitere Funktion von Einrichtung (4) leitet sich aus der Darstellung ab und wird hier nicht weiter beschrieben. Selbstverständlich besteht die Möglichkeit einer Heißreinigung der RO-Anlage ohne Verwendung weiterer Desinfektionsmittel.
Das von der RO-Anlage (2) erzeugte Permeat wird über der Primärseite des Filters (3) zirkuliert. Das von der RO-Steuerung (58) mittels nicht dargestellter Leitfähigkeitsmessung freigegebene Permeat gelangt zur Sekundärseite des Filters (3) und über Permeatfreigabeventil (5) zur Mischeinheit (12).
Eventuell bereits von der RO-Anlage (2) vorgewärmtes Permeat wird über Heizer (9) und Temperaturregler (8,13) auf die erforderliche Spüllösungstemperatur erwärmt. Über Leitung (11) wird das Permeat einer Mischkammer (15) zugeführt, in die mittels Pumpe (23) Konzentrat aus Beutel (26) und Leitung (25), dem Konnektor (24) und geräteseitigem Anschlusskonnektor (22) zugeführt wird.
Dabei ist die Konzentratklappe (20) geöffnet, Detektor (19) meldet "offen", weil Magnet (21) die erforderliche Distanz überschritten hat. Das Konzentratspülventil (17) wird nur bei geschlossener Klappe (20) und entsprechend ausgewählten bzw. voreingestellten Spülprogrammen geöffnet, um den Anschlusskonnektor (22) zu reinigen.
Konzentratbeutel (26) ist mit seinen Aufhängungen (27) in entsprechende Haken der Konzentratbeutelwaage (28) eingehängt.
Die zweite Leitfähigkeits- und Temperaturmessung (16) detektiert aus Gründen der Redundanz die entsprechenden Werte. Die durch Kammer (15) homogen gemischte und temperierte Spülflüssigkeit gelangt über Leitung (29) zu einem zweiten Sterilfilter (30). Fehlerhafte Spülflüssigkeit wird über Bypassventil (31) zum Abfluss (100) verworfen.
Bei geschlossenem Ventil (31) und geöffnetem Spüllösungsfreigabeventil (33) wird die Spülflüssigkeit über den geräteseitigen Spüllösungskonnektor (35) den daran konnektierten Beutelkonnektor (38), Leitung (39) zum fahrbaren Spüllösungsbehälter (40) geleitet, in dem ein steriler Spüllösungsbeutel (82) eingelegt ist. Die Möglichkeit zur Entnahme einer Spüllösungsprobenmenge besteht an Probeentnahme (32).

Der fahrbare Spüllösungsbehälter beinhaltet eine Waage (43), die den jeweiligen Füllstand bzw. das Gewicht der Spülmenge registriert. Ebenfalls ist ein Temperaturfühler (59) so angebracht, dass die Spülflüssigkeitstemperatur indirekt messbar ist.

Bei geschlossener Spüllösungsklappe (36) und bei Auswahl und Einleitung eines entsprechenden Spülprogrammes wird der geräteseitige Konnektor (35) mit steriler Flüssigkeit bzw. Reinigungslösung über Spülabfluss (99) gespült bzw. desinfiziert. Der Test der Filter (3/30) erfolgt bei geschlossenen Klappen (20/36) durch gefilterte Luftzuführung mittels Luftpumpe (6) und kann wahlweise durch Ventilschaltung die Sekundärseite des Filters (3) oder die Primärseite des Filters (30) mit Luft beaufschlagen. Dabei wird die Flüssigkeit partiell durch die Luft verdrängt. Durch den hydrophilen Charakter der Filtermembran wird bei intakter Filtercharakteristik nur ein sehr geringer Druckabfall erfolgen, der mittels Drucksensor (14) und Elektronik (58) registriert bzw. überwacht werden kann.

Durch diesen Test können sowohl die Filter (3/30) als auch die Dichtheit der Klappen (20/ 36) verifiziert bzw. überprüft werden.

Ebenso ist in Figur 1 schematisch ein möglicher Transfer der Spülflüssigkeit zu einem endoskopischen System (57) dargestellt. Druckluftkonnektor (48) kann mittels flexibler Schlauchleitungen (49) an eine hauseigene Druckgasquelle angeschlossen werden.
Zur Gewährleistung eines konstanten Spülflüssigkeitsflusses beinhaltet die Druckregeleinheit (47) einen einstellbaren Druckregler (50), einen Not-Aus mit Pilztaste und Zwangsentlüftung (51), ein manuelles Druckbegrenzungsventil (52), eine Manometeranzeige (53) und einen elektronischen Druckaufnehmer (54), der wie alle Sensoren und Aktoren mittels redundanter Elektronik (58) ausgewertet und dargestellt werden kann.
Das Niederdruck-Regelventil (50) kann nur mittels Werkzeug verstellt werden. Die Druckregeleinheit (47) kann für einen Regelbereich von 0 bis 0,5bar ausgelegt werden und ist für den praktischen Einsatz auf 0,3 bar Förderdruck beispielsweise bei Prostata Operationen eingestellt. Die so geregelte Luft wird über Schlauchverbindung (66) in Druckbehälter (45) eingeleitet.

Die Spülflüssigkeit in Beutel (82) wird durch den zugeführten Druck über Transferanschluss (55) und einem geeigneten Überleitsystem (56) zum endoskopischen System (57) gefördert.

Es versteht sich, dass an System (56) auch andere Einheiten als endoskopische Systeme anschließbar sind.
Zur Ergänzung wird festgestellt, dass an Leitung (55) ein weiterer hier nicht dargestellter Sterilfilter anschließbar wäre.
Ebenso bestünde die Möglichkeit, das geregelte Druckgasmedium direkt im Spüllösungsbeutel (41) einzuleiten.

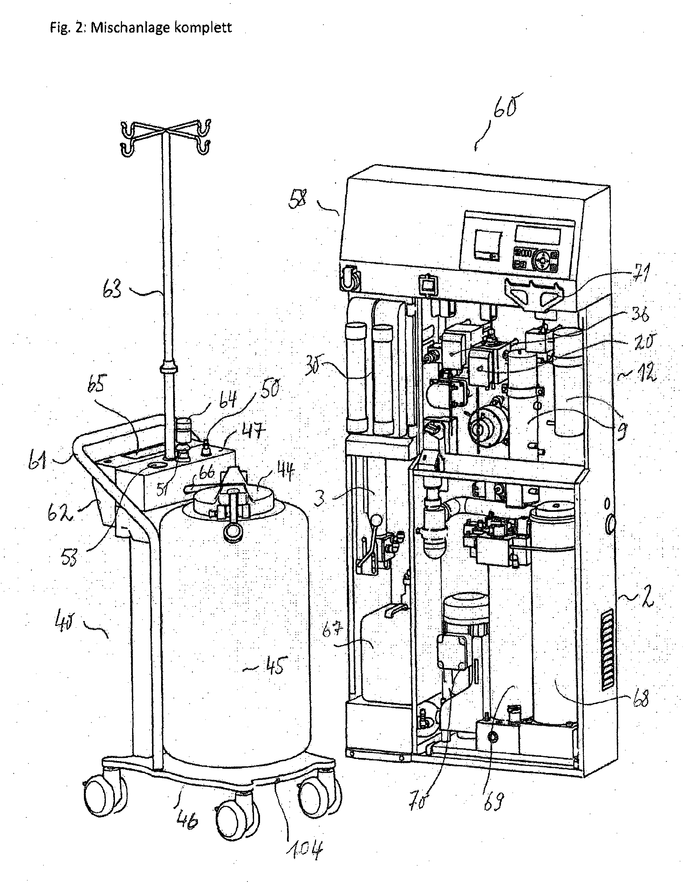

Figur 2 verdeutlicht räumlich die Gesamteinheit einer Mischanlage bzw. Füllstation. Aufgrund der angenommenen räumlich beengten Verhältnisse im Krankenhaus wurde die Füllstation (60) möglichst flach konstruiert um die Durchgänge an Fluren bzw. in Räumlichkeiten nicht zu beeinträchtigen. Dies erfordert eine vertikale Bauweise der RO-Anlage (2) mit Membran (68), Vorlaufbehälter (69) und Pumpe (70). Dargestellt ist auch ein Reinigungskanister (67).
Über der RO-Anlage ist die Mischeinheit (12) angebracht, wobei in dieser Zeichnung lediglich auf die Lage der Konzentratklappe (20), der Spüllösungsklappe (36). dem Heizer (9) und dem Sterilfilter (30) hingewiesen wird, um die Handhabung zu verdeutlichen. Wobei die Klappen hier im geschlossenen Zustand dargestellt sind. Konzentratbeutelwaage (28) ist unterhalb der Elektronik (58) montiert und in Form eines Auslegers (71) mit Aufnahmehaken für den Konzentratbeutel dargestellt.
Die Installation erfolgt wandbündig an geeigneter Stelle mit entsprechendem Höhenabstand zum Fußboden, um Kommunikation - wie später erklärt- und Reinigung zu gewährleisten.

Der fahrbare Spüllösungsbehälter (40) besteht aus einem Transportwagen (46) mit Zug- und Schiebegriff (61), dem Druckbehälter (45), einem Deckel (44), und einer Infusionsstange (63).
Bestandteile des fahrbaren Spüllösungsbehälters (40) sind eine Druckregeleinheit (47), deren Ausgang direkt über eine flexible Schlauchverbindung (66) in den Druckbehälter (45) einmündet und eine Elektronik (62) mit einer Kommunikationsanzeige (65) beispielsweise zur Anzeige des Füllstandes, der Temperatur, Druckluft und anderer relevanter Werte und einer Anzeigeampel (64).

Die Kommunikation zwischen dem Spüllösungsbehälter (40) und der Füllstation (60) erfolgt drahtlos mittels Sensoren im Rollenbereich unterhalb der Bodenplatte (104) des Transportwagens (40).

Die Detektion der Park- bzw. Andockpositionen des Spüllösungsbehälters (40) an die Füllstation (60) ist durch die Lage der vorzugsweisen Infrarotsensoren vorgegeben.

Füllstationsseitig ist auf gleicher Ebene ein korrespondierender Sensor angebracht. Dabei ist durch Auswahl und Lage der Sensoren Andockwinkel und -lage an die Füllstation zu beeinflussen.

Der fahrbare Spüllösungsbehälter (40) kann mit einem Akku und / oder einer Stromversorgung ausgestattet sein; ebenso ist zur Erwärmung bzw. zur verlustfreien Speicherung der erwärmten Spülflüssigkeit eine Isolierung und /oder die Hinzunahme einer Heizung vorzugsweise als Folienheizung möglich. Die Hinzunahme eines internen Kompressors als Druckquelle ist ebenfalls möglich und praktikabel.

Die weiteren Komponenten erklären sich teilweise aus der Darstellung oder werden zu einem späteren Zeitpunkt erläutert. Es versteht sich, dass es sich hier um einen raumsparenden Aufbau der Komponenten handelt, deren Anordnung von der dargestellten abweichen kann bzw. auch in anderen Ausführungsformen vorstellbar ist. Ebenso wurde nicht in allen Punkten Bezug auf die Kennzeichnung genommen.

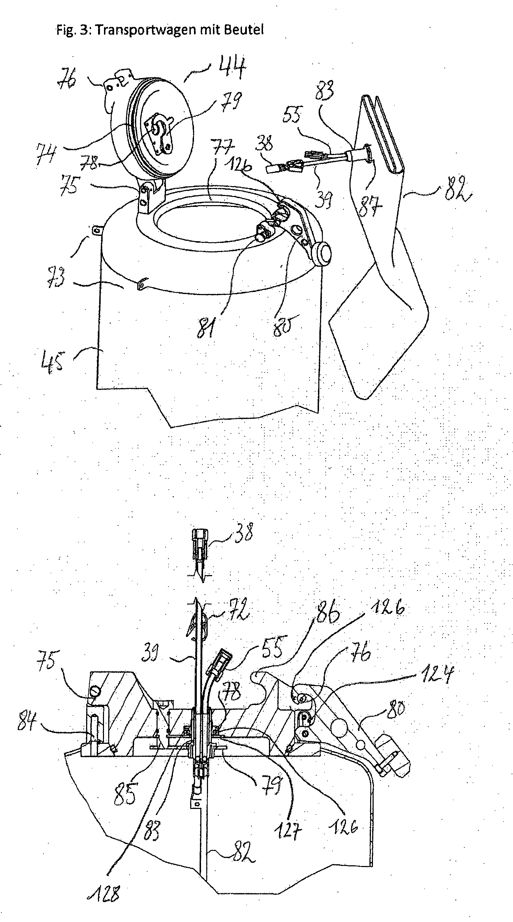

Figur 3 zeigt schematisch den Druckbehälter (45) mit geöffnetem Deckel (44) und einer Konnektoraufnahme (78), durch die der zylindrische Beutelkonnektor (83) eingesteckt wird, und mittels beweglicher Konnektorverriegelung (79) und Haltenut (87) gehalten wird.
Damit es zu einer formschlüssigen Abdichtung mit guten Gleiteigenschaften zwischen Konnektor (83) und Konnektoraufnahmedichtung (78) kommen kann, besteht vorzugsweise Dichtung (78) aus einem Tefloneinsatz (128), der mit einem O-Ring (126) und einer Druckplatte (127) so gegen Konnektor (83) gepresst wird, dass vorgenannte Ziele erreicht werden.
Eine formschlüssige und dichtende Verbindung des Deckels (44) zum Druckbehälter (45)wird einerseits durch Deckeldichtung (74) und dem konischen Dichtungslager (77) in der Druckbehälteröffnung im geschlossenen Zustand hergestellt.
Zum Verschließen zieht Haken (126) die Deckelverriegelung (76) mittels Verriegelungsgriff (80) in Position. Verriegelungssicherung (81) rastet dabei hinter dem Drehgelenk (124) ein.
Deckelklemmscharnier (75) hält Deckel (44) im geöffneten Zustand in aufrechter Position.

Es versteht sich, dass der Beutel (41/82) dazu in den Behälter einzubringen ist.
Zur vertikalen Abstützung sind am Druckbehälter (45) zwei seitliche Führungen (73) angebracht.
Die Druckluftzuführung (66) ist beispielsweise im Scharnierbereich (75) mittels Anschluss (84) angebracht.
Konnektorverriegelung (79) ist im Fehlerfalle von außen über Drehwelle (85) mittels Werkzeug zu öffnen.
Ebenfalls ist in dieser Figur die Füllleitung (39) mit Konnektor (38) dargestellt, die im Füllprozess an Anschluss (35) zu konnektieren ist. Nach dem Füllvorgang kann Klemme (72) geschlossen werden. Zur Differenzierung von Füllleitung (39) und Transferleitung (55) sind diese mit unterschiedlichen Konnektoren bestückt und in unterschiedlichen Längen, wie dargestellt, ausgeführt.

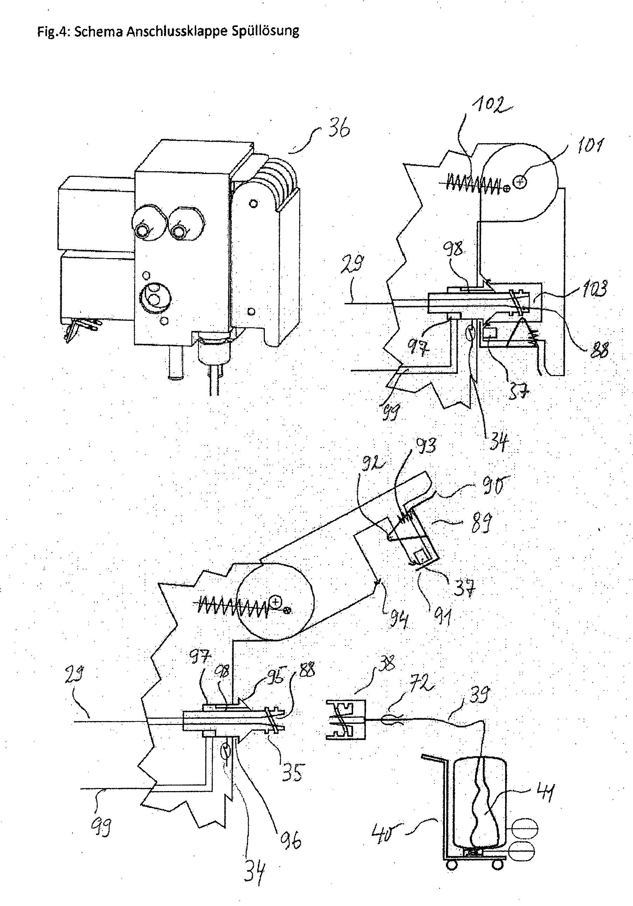

Figur 4 zeigt perspektivisch schematisch die Spüllösungsklappe (36), deren Öffnen, Verschluss, Aushub- und Reinigungsvorgang wie folgt beschrieben wird.
In der Klappe (36) befindet sich ein Magnet (37), der bei geschlossener Klappe einen magnetischen Kontakt (34) aktiviert.
Zum Spülen wird die Klappe (36) geschlossen, sodass der Klappenverriegelungshaken (91) die Klappenverrriegelung (89) in den Verriegelungsbund (96) des Anschlußkonnektors (35) einrastet.
Durch Zurückdrücken der Verriegelung (89) über Drehpunkt (92) mittels Klappenverriegelungsgriff (90) wird die Verriegelungsfeder (93) gestaucht und der Klappenverriegelungshaken (91) gibt dabei den Aushubprozess der Klappe (36) frei.

Die Klappe schwenkt nach oben. Unterstützt wird dies durch Aushubfeder (102), die seitlich des Drehpunktes (101) eingreift.
Zur vollständigen Spülung des Konnektors (35) drückt die Dichtung (94) bei geschlossener Klappe formschlüssig auf den Außenkonus (95) des Konnektors (35). Die Spülflüssigkeit gelangt über Anschluss (29) über den Innenkegel (88) zum Spülraum (103) und von dort über die umfangseitig angebrachten Spülbohrungen (98) des Konnektors (35) in den Ringspalt (97), von dem der Spülabfluss (99) erfolgt.

Aus Gründen der Verwechslungssicherheit für die Anwendung wurde die technische Ausführung der Spüllösungsanschlüsse unterschiedlich zu denen der Konzentratanschlüsse konstruiert.

Unter der Spüllösungsklappe (36) befindet sich ein Spüllösungskonnektor (35) ausgeführt beispielsweise mit einem Innenkegel (88) 1zu 16 und einem zweigängigem Außengewinde 13x8. Am Spüllösungsbeutel (41), der als Einmalartikel ausgeführt ist, befindet sich die Füllleitung (39) mit Einmalkonnektor (38), der als männlicher Konnektor beispielsweise mit frei drehbarer Überwurfmutter mit Innengewinde 13x8 und einem innenliegenden Aussenkegel 1zu16 so ausgestattet ist, dass im gekoppelten Zustand eine formschlüssige, dichtende Verbindung durch die beiden Kegel und Gewinde gewährleistet ist. In die Füllleitung (39) kann eine Schlauchklemme (72) montiert werden.

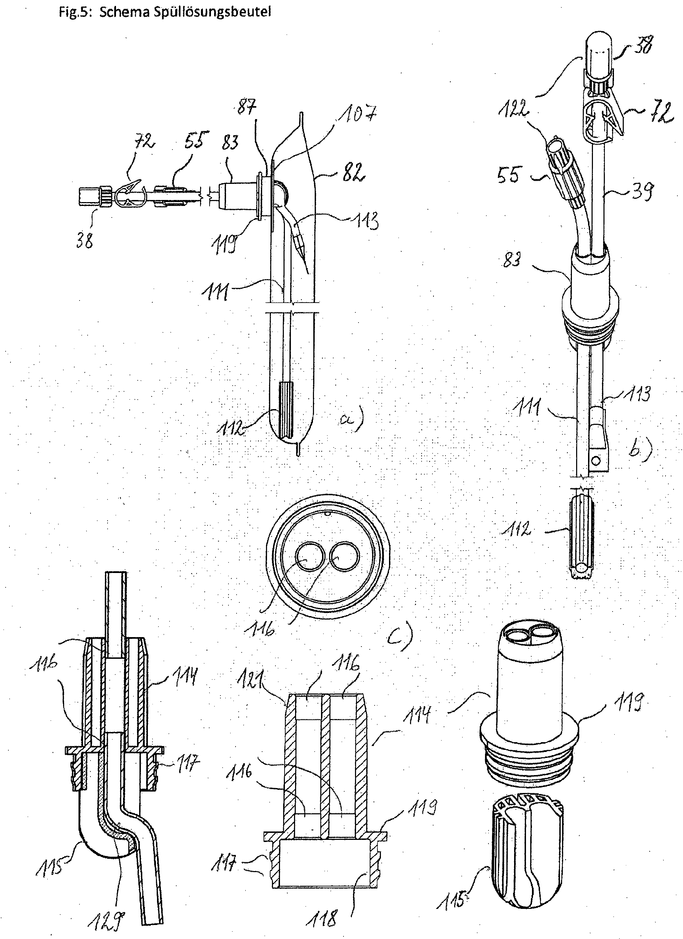

Dabei zeigt die Figur 5 schematisch den Spüllösungsbeutel (82) der aus einem mehrlagigen, toxikologisch unbedenklichen Material - vorzugsweise PE-Folie - besteht.

Der Beutel (82) hat eine rechteckige, geschweißte Kontur in die einseitig eine Beutel- und Konnektoraufnahme (107) kreisförmig in die Folie eingeschweißt ist. Die Konnektoraufnahme (107) ist innenseitig radial mit mindestens 2 Rastzähnen (110) versehen, in die der Konnektor (83) so eingepresst wird, sodass eine formschlüssige, dichtende, unlösbare Verpressung (109) entsteht.

Der Konnektor (83) ist in Figur 5 zweiteilig ausgeführt, wobei auch einteilige, ähnliche Geometrien im Rahmen der Erfindung möglich sind. In der dargestellten Version besteht Konnektor (83) aus einem Vorderteil (114), dessen wesentliche Bestandteile die Schlauchklebestellen (116), in die innerhalb und außerhalb die Transfer- und Füllleitungen vorzugsweise eingeklebt sind, sowie der Bund (119) für die Haltenut (87) und Rastzähne (117).
Zum leichteren Einführen in die Konnektor-Aufnahme (78) des Deckels (44) beinhaltet das Vorderteil (114) einen Anschnitt (121). Rückwärtig ist eine Aufnahme (118) für den Knickschutz (115) der inneren Leitungen (111, 113) vorgesehen.

Im eingebauten Zustand wird der Beutelkonnektor (83) vertikal mit Konnektorverriegelung (79) in Haltenut (87) befestigt.

Bei geschlossenem Deckel (44) erfolgt eine Verdrehung des Beutels (82) um ca. 90° gegen den Konnektor (83) und damit auch eine Lageveränderung der inneren Transfer- (111) und Füllleitung (113). Der Knickschutz (115) beinhaltet Schlauchführungen (129), die ein eventuelles Abknicken derselben verhindern sollen.

Die Transferleitung (111) schließt am unteren Ende mit einem Schlauchgewicht (112) ab, welches an allen Außenseiten Konturen aufweist, um zum einen eine vollständige Entleerung des Beutels bei zusammengedrückter Folie zu gewährleisten und um einem eventuellen Auftrieb der Transferleitung (111) entgegenzuwirken.

Zur Vermeidung einer Berührungskontaminierung können die Konnektoren (55) und (38) Schutzkappen (122) mit Öffnungen für die Begasungen zwecks Sterilität beinhalten.

Die Folien des Beutels (82) sind umfangsseitig so verschweißt, dass ein Schweißsteg von min. größer 2 mm vorliegt um eine Ruptur zu vermeiden.

Bereits dargestellt in Figur 3 ist eine mögliche Faltung des Beutels (82), die ein leichteres Einlegen in die Druckbehälteröffnung (77) ermöglicht.

Wobei Beutel (82) auch in andere als in der Beschreibung dargestellten Kontur bzw. Form ausführbar ist. So ist es z.B. möglich die beiden Anschlüsse des Konnektors (83) stirnseitig, also an der Oberseite des Beutels (82) anzubringen, bzw. ganz auf den Konnektor (83) zu verzichten und die Anschlüsse (39/55) direkt oberseitig einzuschweißen. Es ist dann eine andere formschlüssige Abdichtung einzusetzen. Darüber ist bei Einsatz einer zusätzlichen in den Figuren nicht dargestellten Pumpe eine Zirkulation der im Beutel befindlichen Flüssigkeit über die beiden Anschlüsse des Konnektors (83) durchführbar, um Homogenität, Temperatur oder eine bereits im Beutel befindlichen Flüssigkeit zu verbessern bzw. aufzubereiten. Die Anschlüsse sind dabei der Strömungsrichtung der Pumpe bzw. dem Zweck entsprechend zu konnektieren.

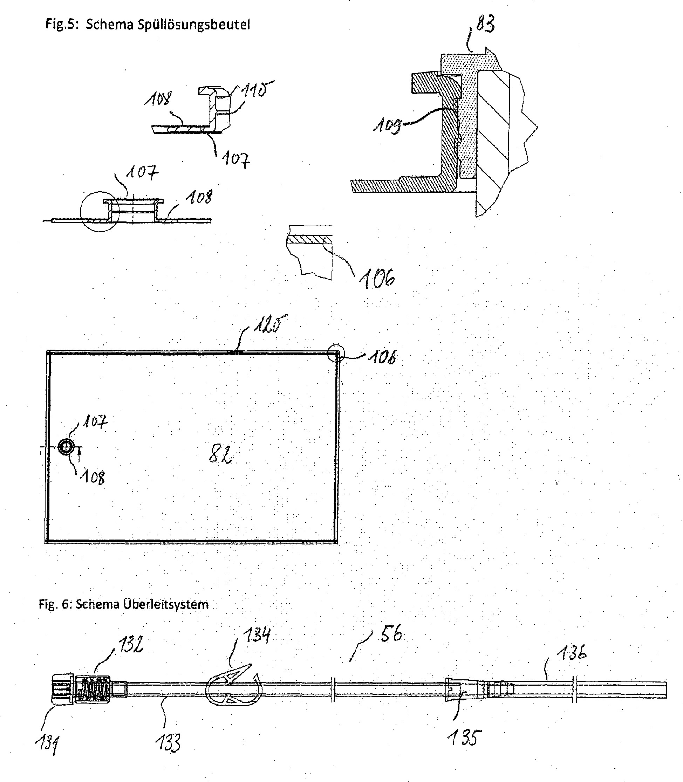

Figur 6 zeigt das Schema eines Überleitsystems welches mit seinem Konnektor (132) an den Transferanschluss (55) adaptiert werden kann und mit dem konischen Anschlußstück (134) bzw. einem daran folgenden Silikonschlauchstück (136) an ein in der Chirurgie übliches Endoskopie- oder auch Spülsystem anzuschließen ist.
Der Konus (135) ermöglicht die Aufnahme unterschiedlicher Silikonschlauchdurchmesser (136) um unterschiedliche, in der Chirurgie gebräuchliche System anzuschließen.
Die Schlauchklemme (134) kann vorteilhafterweise auch als Rollenklemme zur Regelung des Spülflusses ausgeführt sein.
Die Länge des Schlauches (133) ist frei wählbar und in ihrem Durchmesser dem gewünschten Spülfluss anzupassen, wobei für das gezeigte System ein Schlauchdurchmesser (133) von ca. 7 mm gewählt wurde, welches in Konnektor (132) eingeklebt ist.
Aus Gründen der Sterilität wurde Anschluss (132) mit einer begasbaren Kappe (131) bestückt.

**Legende**

| | |
|---|---|
| 1. | Vorfiltration |
| 2. | RO-Anlage |
| 3. | Permeat Ultra-/Sterilfilter |
| 4. | Desinfektionseinheit |
| 5. | Permeatfreigabeventil |
| 6. | Luftdruckzuführung, Luftpumpe |
| 7. | Luftansaugfilter |
| 8. | Temperaturregler |
| 9. | Heizer |
| 10. | Übertemperaturschutz |
| 11. | Permeatversorgungsleitung |
| 12. | Mischeinheit |
| 13. | Temperaturregler / -anzeige |
| 14. | Drucksensor |
| 15. | Mischkammer |
| 16. | Redundante Leitfähigkeitsmessung / Temperaturanzeige |
| 17. | Konzentratspülventil |
| 18. | Spülleitung |
| 19. | Konzentratklappendetektor |
| 20. | Konzentratklappe |
| 21. | Magnet |
| 22. | Konzentratbeutelanschlusskonnektor mit zweigängigem Innengewinde und innenliegendem Außenkegel |
| 23. | Konzentratpumpe |
| 24. | Konzentratbeutelkonnektor mit Brechkonus mit 2 gängigen Außengewinden und Innenkonus |
| 25. | Konzentratbeutelanschlus |
| 26. | Konzentratbeutel |
| 27. | Aufhängung Konzentratbeutel |
| 28. | Konzentratbeutelwaage |
| 29. | Spüllösungsleitung |
| 30. | Sterilfilter 2 |
| 31. | Spüllösungsbypassventil |
| 32. | Probeentnahme |
| 33. | Spüllösungsfreigabeventil |
| 34. | Spüllösungsklappendetektor |
| 35. | Spüllösungskonnektor mit Innenkegel und zweigängigem Außengewinde |
| 36. | Spüllösungsklappe |
| 37. | Magnet |
| 38. | Spüllösungsbeutelkonnektor mit Außenkonus und Innengewinde |
| 39. | Spüllösungsfüllleitung |
| 40. | Fahrbarer Spüllösungsbehälter |
| 41. | |
| 42. | |
| 43. | Spüllösungsbehälter-Waage |
| 44. | Deckel |
| 45. | Druckbehälter |
| 46. | Transportwagen |
| 47. | Druckregeleinheit |
| 48. | Druckluftkonnektor |
| 49. | Schlauchverlängerung |
| 50. | Druckregler |
| 51. | Not-Aus |
| 52. | Druckbegrenzungsventil |
| 53. | Druckmanometer |
| 54. | Drucksensor |
| 55. | Transferanschluss mit zweigängigem Außengewinde, Innenkonus und Verschlussklappe |
| 56. | Überleitsystem |
| 57. | OP-Anwendung |
| 58. | Elektronik |
| 59. | Temperaturfühler |
| 60. | Füllstation |
| 61. | Zug- und Schiebegriff |
| 62. | Elektronik für Transportwagen |
| 63. | Infusionsstange |
| 64. | Anzeige Ampel |
| 65. | Kommunikationsanzeige für Druck, Temperatur, Füllstand |
| 66. | Druckluft-Schlauchverbindung |
| 67. | Desinfektions- / Reinigungsmittelkanister |
| 68. | RO-Membran |
| 69. | Vorlaufbehälter |
| 70. | Pumpe mit Antrieb |
| 71. | Konzentratwaagen-Ausleger mit Beuteleinhänghaken |
| 72. | Schlauchklemme |
| 73. | Seitliche Führung Druckbehälter |
| 74. | Deckeldichtung |
| 75. | Deckelklemmscharnier |
| 76. | Deckelverriegelungsbolzen |
| 77. | Druckbehälteröffnung mit konischem Dichtungslager |
| 78. | Konnektoraufnahme mit innenliegender vorgespannter Gleitrichtung |
| 79. | Konnektorverriegelung |
| 80. | Verriegelungsgriff mit Haken |
| 81. | Verriegelungssicherung |
| 82. | Spüllösungsbeutel |
| 83. | Beutelkonnektor |
| 84. | Druckluftzuführung |
| 85. | Verriegelungsdrehwelle mit außenliegendem Innensechskant |
| 86. | Aushebegriff |
| 87. | Haltenut |
| 88. | Innenkegel Spüllösungskonnektor |
| 89. | Klappenverriegelung |
| 90. | Klappenverriegelungsgriff |
| 91.. | Klappenverriegelungshaken |
| 92. | Klappenverriegelungsdrehpunkt |
| 93. | Klappenverriegelungsfeder |
| 94. | Klappendichtung |
| 95. | Dichtungsgegenlager |
| 96. | Verriegelungsbund |
| 97. | Ringspalt Spülfluss |
| 98. | Spülbohrungen |
| 99. | Spülabfluss |
| 100. | Abfluss |
| 101. | Drehwelle Spüllösungsklappe |
| 102. | Aushubfeder |
| 103. | Spülraum |
| 104. | Bodenplatte fahrbarer Spüllösungsbehälter |
| 105. | Rollen |
| 106. | Umfangsseitige Beutelschweißnaht |
| 107. | Konnektoraufnahme |
| 108. | Kreisförmige Folienanschweißstelle |
| 109. | Konnektorverpressung |
| 110. | Rastzähne an Konnektoraufnahme |
| 111. | Innere Transferleitung |
| 112. | Gewicht |
| 113. | Innere Füllleitung mit optionalem Rückschlagventil |
| 114. | Beutel-Konnektor Vorderteil |
| 115. | Beutel-Konnektor Knickschutz |
| 116. | Schlauchklebestellen |
| 117. | Rastzähne Beutelaufnahme |
| 118. | Aufnahme Knickschutz |
| 119. | Bund Haltenut |
| 120. | Beutelbeschriftung |
| 121. | Konnektor-Einführfase |
| 122. | Sterilität Verschlusskappen |
| 123. | Drehpunkt für Drehgelenk |
| 124. | Drehgelenk |
| 125. | Phase Drehgelenk |
| 126. | O-Ring |
| 127. | Druckplatte |
| 128. | Tefloneinsatz |
| 129. | Schlauchführungen |
| 130. | Haken Deckelverriegelung |
| 131. | Begasungskappe mit Außengewinde 13x8 |
| 132. | Patientenkonnektor mit Überwurfmutter, Innengewinde 13x8 und Außenkegel 1/16 |
| 133. | Schlauch ca. 7mm |
| 134. | Schlauchklemme |
| 135. | Konus geriffelt (5-10mm) |
| 136. | Silikonschlauchstück |

## Patentansprüche

1. Anordnung zur Speicherung und Verabreichung von Spüllösungen, vorzugsweise im Medizinbereich wie für allgemeine und endoskopische Operationen, mit einem Spüllösungsbeutel, der in einem starren Behälter (45) angeordnet ist,
**dadurch gekennzeichnet,**
**dass** der starre Behälter (45) einen schwenkbaren Deckel (44) mit einer Konnektoraufnahme (78) aufweist, durch die ein Beutelkonnektor (83) durchgesteckt wird, der mittels einer beweglichen Konnektorverriegelung (79) und einer Haltenut (87) an dem Beutelkonnektor (83) befestigbar ist,
**dass** durch den Beutelkonnektor (83) eine Füllleitung (39) verläuft, die mit einem Anschlusskonnektor (38) versehen ist, der mit einem Anschlusskonnektor (35) einer Mischeinheit (12) verbindbar ist,
**dass** der Anschlusskonnektor (22) der Mischeinheit von einer schwenkbaren Konzentratklappe (20) in deren geschlossenen Zustand so überdeckt ist, dass ein dichter Spülraum (103) um den Anschlusskonnektor (22) gebildet ist, und dass eine Spülflüssigkeitsleitung in den Anschlusskonnektor (22) der Mischeinheit einmündet, so dass Spülflüssigkeit in den Spülraum (103) förderbar ist, die den Anschlusskonnektor (22) innen und außen vollständig reinigen kann.

2. Anordnung nach Anspruch 1,
**dadurch gekennzeichnet,**
**dass** der starre Behälter als Druckbehälter (45) ausgebildet ist.

3. Anordnung nach Anspruch 1 bis 2,
**dadurch gekennzeichnet,**
**dass** der Spüllösungsbeutel (82) aus einer mehrlagigen Folie gebildet ist, die vorzugsweise aus PE besteht und an den Rändern verschweißt ist.

4. Anordnung nach den Ansprüchen 1 bis 3,
**dadurch gekennzeichnet,**
**dass** der Beutelkonnektor (83) ein Vorderteil (114) hat, in den die Füllleitung (39) und die Transferleitung (55) eingeklebt sind.

5. Anordnung nach den Ansprüchen 1 bis 5,
**dadurch gekennzeichnet,**
**dass** der Beutelkonnektor (83) eine außen umlaufende Nut (87) aufweist, in die ein Riegel (79) des Deckels (44) eingreift.

6. Anordnung nach den Ansprüchen 1 bis 5,
**dadurch gekennzeichnet,**
**dass** in eine Seitenfläche des Spüllösungsbeutels (82) eine im wesentlichen hülsenförmige Konnektoraufnahme (107) eingeschweißt ist, die innenseitig mit Rastzähnen (110) versehen ist,
**dass** der Beutelkonnektor (83) einen rückwärtigen hülsenförmigen Abschnitt mit Rastzähnen (117) hat, der so in die Konnektoraufnahme (107) einpressbar ist, dass eine formschlüssige, dichtende und unlösbare Verpressung (109) entsteht.

7. Anordnung nach den Ansprüchen 1 bis 6,
**dadurch gekennzeichnet,**
**dass** in dem rückwärtigen hülsenförmigen Abschnitt des Beutelkonnektors (83) ein Knickschutz (115) mit Schlauchführungen (129) angeordnet ist.

8. Anordnung nach den Ansprüchen 1 bis 7,
**dadurch gekennzeichnet,**
**dass** an dem Ende der Transferleitung (11) in dem Spüllösungsbeutel (82) ein Schlauchgewicht (112) befestigt ist, und
**dass** das Schlauchgewicht (112) an der Außenseite eine längs gerichtete rillenförmige Kontur (112) aufweist.

9. Anordnung nach den Ansprüchen 1 bis 8,
**dadurch gekennzeichnet,**
**dass** eine Pumpe angeordnet ist, die mit der Füllleitung (39) und der Transferleitung (55) verbindbar ist, um die in dem Spüllösungsbeutel (83) befindliche Flüssigkeit zu zirkulieren.

10. Anordnung nach den Ansprüchen 1 bis 9,
ferner **gekennzeichnet**
**durch** einen Transportwagen (46) für den Druckbehälter (45), der eine Druckregeleinheit (47) für den Druck in dem Druckbehälter (45) aufweist.

## Claims

1. A system for storing and administering flushing solutions, preferably in the medical field such as for general and endoscopic operations, with a flushing solution bag, which is arranged in a rigid container (45), **characterised in that** the rigid container (45) includes a pivotable cover (44) with a connector receptacle (78), through which a bag connector (83) is pushed, which is fastenable to the bag connector (83) by means of a movable connector lock (79) and a retaining groove (87), that extending through the bag connector (83) there is a filling conduit (39), which is provided with a connector (38), which is connectable to a connector (35) of a mixing unit (12), that the connector (22) of the mixing unit is covered by a pivotable concentrate flap (20) in its closed state so that a sealed flushing space is defined around the connector (22) and that a flushing liquid conduit communicates with the connector (22) of the mixing unit so that flushing liquid may be conveyed into the flushing space (103), which can completely clean the connector (22) internally and externally.

2. A system as claimed in Claim 1, **characterised in that** the rigid container is constructed in the form of a pressure container (45).

3. A system as claimed in Claims 1 to 2, **characterised in that** the flushing solution bag (82) is constituted by a multi-layer film, which preferably consists of PE and is welded at its edges.

4. A system as claimed in Claims 1 to 3, **characterised in that** the bag connector (83) has a front portion (114), in which the filling conduit (39) and the transfer conduit (55) are secured by adhesive.

5. A system as claimed in Claims 1 to 5, **characterised in that** the bag connector (83) includes an outer peripheral groove (87), into which a latch (79) on the cover (44) engages.

6. A system as claimed in Claims 1 to 5, **characterised in that** welded into a side surface of the flushing solution bag (82) there is a substantially sleeve-shaped connector receptacle (107), which is provided internally with locking teeth (110), that the bag connector (83) has a rear, sleeve-shaped section with locking teeth (117), which may be pressed into the connector receptacle (107) so that a positive, sealed and non-releasable compression (109) is produced.

7. A system as claimed in Claims 1 to 6, **characterised in that** arranged in the rear, sleeve-shaped section of the bag connector (83) there is a kink preventer (115) with hose guides (129).

8. A system as claimed in Claims 1 to 7, **characterised in that** fastened at the end of the transfer conduit (11) in the flushing solution bag (82) there is a hose weight (112) and that the hose weight (112) has on its outer surface a longitudinally directed, groove-shaped contour (112).

9. A system as claimed in Claims 1 to 8, **characterised in that** a pump is arranged, which is connectable to the filling conduit (39) and the transfer conduit (55) in order to circulate the liquid located in the flushing solution bag (83).

10. A system as claimed in Claims 1 to 9, **characterised by** a transport carriage (46) for the pressure container (45), which includes a pressure control unit (47) for the pressure in the pressure container (45).

## Revendications

1. Système de stockage et de distribution de solutions de rinçage, de préférence dans le domaine médical tel que pour les opérations générales et endoscopiques, avec une poche de solution de rinçage qui est disposée dans un récipient rigide (45),
**caractérisé en ce**
**que** le récipient rigide (45) présente un couvercle pivotant (44) comportant un logement de connecteur (78) au travers duquel un connecteur de poche (83) est enfiché, qui peut être fixé au moyen d'un verrouillage de connecteur mobile (79) et d'une rainure de retenue (87) au connecteur de poche (83),
**qu'**une conduite de remplissage (39) parcourt le connecteur de poche (83), laquelle conduite est dotée d'un connecteur de raccordement (38) qui peut être raccordé à un connecteur de raccordement (35) d'une unité de mélange (12),
**que** le connecteur de raccordement (22) de l'unité de mélange est recouvert d'un clapet de concentré pivotant (20), lorsqu'il est à l'état fermé, de telle sorte qu'un espace de rinçage étanche (103) est formé autour du connecteur de raccordement (22) et qu'une conduite de liquide de rinçage débouche dans le connecteur de raccordement (22) de l'unité de mélange, de sorte que du liquide de rinçage puisse être acheminé dans l'espace de rinçage (103), qui peut nettoyer complètement l'intérieur et l'extérieur du connecteur de raccordement (22).

2. Système selon la revendication 1,
**caractérisé en ce**
**que** le récipient rigide est conçu comme un récipient sous pression (45).

3. Système selon la revendication 1 à 2,
**caractérisé en ce**
**que** la poche de solution de rinçage (82) est formée d'un film de plusieurs couches qui consiste de préférence en PE et est soudé sur les bords.

4. Système selon les revendications 1 à 3,
**caractérisé en ce**
**que** le connecteur de poche (83) a une partie avant (114) dans laquelle la conduite de remplissage (39) et la conduite de transfert (55) sont collées.

5. Système selon les revendications 1 à 5,
**caractérisé en ce**
**que** le connecteur de poche (83) présente une rainure périphérique extérieure (87) dans laquelle un verrou (79) du couvercle (44) est en prise.

6. Système selon les revendications 1 à 5,
**caractérisé en ce**
**que**, sur une surface latérale de la poche de solution de rinçage (82), est soudé un logement de connecteur (107) sensiblement en forme de gaine, qui est doté de dents d'encliquetage (110) du côté intérieur,
**que** le connecteur de poche (83) a un segment en forme de gaine sur l'arrière, doté de dents d'encliquetage (117) qui peut être inséré dans le logement de récepteur (107) de telle sorte qu'il se forme une insertion (109) par liaison de forme, étanche et indissociable.

7. Système selon les revendications 1 à 6,
**caractérisé en ce**
**que**, dans le segment en forme de gaine sur l'arrière du connecteur de poche (83), un dispositif anti-pliure (115) comportant des guides de tubulure (129) est aménagé.

8. Système selon les revendications 1 à 7,
**caractérisé en ce**
**qu'**à la fin de la conduite de transfert (11) dans la poche de solution de rinçage (82), un poids de tuyau (112) est fixé, et
**que** le poids de tuyau (112) présente, sur le côté extérieur, un contour en forme de rayure (112) orienté longitudinalement.

9. Système selon les revendications 1 à 8,
**caractérisé en ce**
**qu'**une pompe qui peut être reliée à la conduite de remplissage (39) et à la conduite de transfert (55) est aménagée pour faire circuler le liquide se trouvant dans la poche de solution de rinçage (83).

10. Système selon les revendications 1 à 9,
**caractérisé en outre**
**par** un chariot de transport (46) pour le récipient sous pression (45) qui présente une unité de réglage de la pression (47) pour la pression dans le récipient sous pression (45).
